# FASCICULE DE BREVET EUROPEEN

(11) **EP 2 542 287 B1**
(45) Date de publication et mention de la délivrance du brevet: **30.12.2015**
(21) Numéro de dépôt: 11708909.4
(22) Date de dépôt: 09.02.2011
(51) Int. Cl.: A61M 16/00, A61B 5/08, A61B 5/087

(54) **DISPOSITIF MÉDICAL DE SUIVI DE L'OBSERVANCE DE PATIENTS APNÉIQUES**
MEDIZINISCHE VORRICHTUNG ZUR ÜBERWACHUNG DER KOMPLIANZ VON PATIENTEN MIT APNOE
MEDICAL DEVICE FOR MONITORING THE COMPLIANCE OF PATIENTS WITH APNEA

(30) Priorité: 01.03.2010 FR 1051427
(43) Date de publication de la demande: 09.01.2013
(73) Titulaire: L'Air Liquide Société Anonyme pour l'Etude et l'Exploitation des Procédés Georges Claude, 75007 Paris (FR)
(72) Inventeur: CARLOS, Géraldine, F-92200 Neuilly Sur Seine (FR); DESFORGES, Daniel, F-75116 Paris (FR); WEBER, Claude, F-91490 Milly La Forêt (FR)
(74) Mandataire: Pittis, Olivier
(86) Numéro de dépôt international: PCT/FR2011/050266
(87) Numéro de publication internationale: WO 2011/107684

(56) Documents cités:
- EP-A1- 2 017 586
- US-A1- 2004 123 866
- US-A1- 2004 187 871

## Description

L'invention porte sur un dispositif de suivi de l'observance d'un traitement de l'apnée obstructive du sommeil (AOS).

Le syndrome d'apnée obstructive du sommeil (SAOS) est un trouble répandu affectant des millions d'adultes et d'enfants, qui se caractérise par une obstruction des voies aériennes supérieures, laquelle peut provoquer des ronflements ou des arrêts de la respiration durant le sommeil.

L'obstruction qui a lieu pendant le sommeil a en fait deux causes principales : le manque de tonus musculaire et la pesanteur. En effet, la présence excessive de tissus dans les voies aériennes supérieures et des déformations anatomiques aggravent les conséquences de ces facteurs. Ainsi, pendant le sommeil, particulièrement pendant le sommeil paradoxal, le corps se détend et les tissus musculaires, comme la langue et le voile du palais par exemple, perdent de leur rigidité. Par ailleurs, lorsque l'on dort en position allongée, l'effet de la pesanteur pousse ces tissus vers le fond de la gorge, ce qui ferme les voies aériennes supérieures.

Lorsqu'ils bloquent complètement les voies aériennes supérieures, ces tissus empêchent la personne de respirer au risque de provoquer une asphyxie. Mais, en général, la personne se réveille assez pour reprendre le contrôle de ses voies aériennes supérieures et respirer, avant de se rendormir. Chez une personne souffrant du SAOS, ce phénomène peut se produire des dizaines voire des centaines de fois par nuit mais habituellement la personne ne s'en rappelle pas au réveil.

Or, chaque obstruction prive le corps d'oxygène et le force donc à garder du dioxyde de carbone (CO₂) qu'il expulserait normalement lors des phases d'expiration. Il s'ensuit que l'équilibre gazeux du sang est perturbé et le corps exposé à un environnement " toxique ". Lorsque le corps "signale " qu'il a besoin de plus d'oxygène, le cerveau réveille le dormeur, la respiration reprend et la personne se rendort jusqu'à la prochaine obstruction. Ces obstructions entraînent également une augmentation du rythme cardiaque et de la pression artérielle, et éventuellement affaiblissent la capacité de réaction " automatique " du corps, ce qui se traduit par des apnées et hypopnées de plus en plus sévères.

Les micro-réveils cycliques que connaissent les personnes atteintes du SAOS affectent la qualité de leur sommeil. Les symptômes de la privation de sommeil chez les personnes atteintes du SAOS sont notamment une somnolence diurne excessive, un manque de concentration, une mauvaise mémoire, voire même un état dépressif. L'hypertension et la baisse du taux d'oxygène sanguin sont des symptômes fréquents pour les personnes qui souffrent d'apnée du sommeil, mais ce sont des symptômes difficiles à détecter. Il existe par ailleurs d'autres symptômes plus faciles à identifier tels que somnolence diurne, ronflements, apnées ou respiration irrégulière pendant le sommeil, perte de concentration.

Un traitement efficace du SAOS consiste à appliquer une pression positive d'air aux voies respiratoires du patient. La pression d'air agit comme un "coussin d'air" qui maintient ouvertes les voies respiratoires supérieures et empêche les apnées. Pour ce faire, on utilise typiquement un appareil de type à PPC (pour Pression Positive Continue) ou CPAP (en anglais pour *Continuons Positive Airway Pressure*) qui délivre de l'air légèrement pressurisé aux voies aériennes du patient, via une conduite souple, appelé circuit patient, reliée à un masque respiratoire nasal ou facial.

Le traitement par PPC est un traitement efficace pour les patients atteints du SAOS si son application est bien suivie et il peut conduire à une nette amélioration de la qualité de vie du patient. En revanche, les effets du traitement sont négligeables, voire nuls, si le patient n'observe pas son traitement pendant au moins 4 h/nuit.

Connaître l'observance des patients, c'est-à-dire mesurer le temps réel pendant lequel ils suivent leur traitement est donc essentiel. De même, connaître en temps « réel » l'efficacité du traitement est une aide précieuse pour ajuster la prescription du traitement par le médecin traitant.

Les systèmes d'observance et d'efficacité actuels sont intégrés aux machines de traitement du SAOS et les résultats ne sont connus qu'en temps différés, c'est-à-dire typiquement entre 1 et 3 mois après passage au domicile du patient d'un personnel de suivi du traitement qui récupère les données enregistrées sur carte mémoire ou via une communication avec un ordinateur.

On comprend que ce délai n'est pas idéal si l'on veut obtenir un suivi en temps réel ou quasi réel du patient, de manière à pouvoir réagir rapidement si l'on s'aperçoit que le patient ne suit pas ou alors mal son traitement, et/ou que le traitement n'est pas adapté.

En fait, il n'existe aujourd'hui aucun dispositif médical de traitement de l'apnée du sommeil permettant de répondre pleinement à l'objectif fixé. En effet, les systèmes de mesure d'observance et d'efficacité existants sont toujours intégrés aux dispositifs de traitement en utilisant des informations internes à ces dispositifs, notamment mesure de temps de traitement, mesure de quantité d'apnées et hypopnées par unité de temps, mesure du temps de ronflement, temps de fuite du circuit respiratoire...

Ces systèmes mémorisent les données d'observance et d'efficacité et celles-ci doivent être récupérées par lecture de la mémoire de l'appareillage, c'est-à-dire sur une mémoire interne ou une carte mémoire amovible, via un ordinateur ou une liaison filaire ou une transmission Radio Fréquence (RF) à un serveur distant dépendant du constructeur du dispositif de traitement.

Les machines de traitement actuelles ont donc une fonction d'observance intégrée mais totalement spécifique à chaque machine, ce qui n'est pas pratique. De plus, le format du rapport d'observance est également spécifique et il n'est donc pas toujours aisé d'interpréter des données d'observance provenant d'une machine par rapport à une autre.

Par ailleurs, le document US-A-6910481 propose un système communiquant et indépendant des dispositifs de traitement. Toutefois, ce système ne permet pas de suivre l'efficacité du traitement du SAOS.

Le document EP 2 017 586 A1 divulgue les caractéristiques du préambule de la revendication 1.

Le problème à résoudre est dès lors de proposer un dispositif de suivi qui permet de connaître et de contrôler à distance l'observance et l'efficacité d'un traitement par PPC effectué à domicile, c'est-à-dire chez le patient, et ce, quelle que soit la machine de traitement utilisée.

En d'autres termes, le dispositif doit permettre de déterminer si un patient suit bien le traitement qui lui a été prescrit, d'enregistrer ces données et de les transmettre localement et/ou à distance, de préférence sans liaison filaire, afin de pouvoir alerter le patient et/ou le personnel soignant et/ou le centre de soins auquel il est rattaché en cas de non suivi, de mauvais suivi ou de non efficacité du traitement, et par ailleurs être utilisable en association avec n'importe quelle machine de traitement par PPC.

La solution de l'invention est un dispositif de suivi de l'observance d'un traitement de l'apnée obstructive du sommeil (AOS) comprenant :
- un passage de gaz comprenant une entrée et une sortie, auquel est raccordé un dispositif de détermination de débit pour déterminer le débit du gaz circulant entre l'entrée et la sortie,
- deux capteurs de pression absolue ou un capteur de pression relative pour déterminer la différence de pression entre au moins une partie du passage et l'atmosphère extérieure,
- des moyens de stockage de données pour mémoriser au moins l'une desdites données,
- des moyens d'émission aptes à et conçus pour transmettre au moins l'une desdites données, et
- au moins la portion de passage de gaz située entre l'entrée et la sortie, les moyens de stockage de données et les moyens d'émission sont compris dans un boîtier,
caractérisé en ce que :
- il comprend des moyens de traitement comprennent au moins un microcontrôleur et un algorithme,
- ledit dispositif de détermination de débit et ledit ou lesdits capteurs de pression sont reliés aux moyens de traitement,
- lesdits moyens de traitement étant aptes à et conçus pour :
   i) traiter les valeurs de débit et de pression déterminées par ledit dispositif de détermination de débit et ledit ou lesdits capteurs et en déduire au moins une donnée de durée de traitement journalier et au moins une donnée d'efficacité de traitement de l'AOS, où :
      l adite donnée d'efficacité de traitement étant déterminée à partir du nombre d'événements respiratoires détectés choisis parmi les apnées, les hypopnées, les limitations de débit et les ronflements, pendant une période de temps T, par l'algorithme des moyens de traitement à partir des mesures de pression et de débit opérées pendant la période de temps T considérée, et
      l adite durée de traitement étant déterminée par l'algorithme, pour un temps donné divisé en périodes d'une durée fixée (T) en :
         a) déterminant si, pendant une partie des périodes de temps (T) données, le patient suit son traitement,
         b) comptabilisant le nombre de périodes pendant lesquelles le patient a suivi son traitement et
         c) calculant la durée de suivi du traitement, ladit durée du traitement (Dt) correspondant au nombre (N) de périodes de traitement multiplié par la durée de la période (T) considérée,
   ii) détecter la respiration du patient et la pression minimale de traitement par traitement des signaux de débit (Q) et de pression (P) mesurés par ledit dispositif de détermination de débit et ledit ou lesdits capteurs de pression, l'algorithme vérifiant sur une période de temps donnée, à partir des mesures de pression (P) et de débit (Q), si la pression (Pmoyenne) dans le circuit du patient est suffisamment élevée par rapport à la pression atmosphérique (Patm), et si l'écart-type du signal de débit (E.T.Q) dépasse un seuil fixé (E.T.limite), et en déduisant si le traitement a eu lieu ou non, pendant la période de temps (T) considérée,
      - et les moyens d'émission comprennent un système émetteur sans fil et une antenne permettant d'assurer une transmission sans fil des données.

Dans le cadre de l'invention :
- l'observance est la durée d'utilisation du traitement, par exemple le nombre d'heures ou de minutes par nuit, le nombre de jours par semaine ou par mois, le nombre d'heures ou de minutes moyen par nuit ou par jour, le nombre de jours pour lesquels la durée du traitement a été supérieur à 4h, le nombre d'heure de fonctionnement par jour de la machine de traitement par PPC.
- l'efficacité du traitement est une indication du nombre résiduel d'apnées obstructives et centrales, hypopnées, limitations de débit, ronflements et/ou de la pression moyenne de gaz appliquée, ainsi que des fuites non-intentionnelles de gaz éventuelles s'étant produits durant la période de traitement considérée.

Selon le cas, le dispositif de l'invention peut comprendre l'une ou plusieurs des caractéristiques suivantes :
- les moyens de stockage de données comprennent au moins une puce mémoire ou une carte mémoire, de préférence enfichable, par exemple une carte SD ou analogue.
- les moyens d'émission comprennent un système émetteur sans fil de type radiofréquence, bluetooth, Zigbee, wifi, GSM ou GPRS, et une antenne permettant d'assurer une transmission sans fil des données adaptée au type d'émetteur et insérée dans le boîtier.
- il comprend en outre des moyens d'alimentation en courant électrique raccordés électriquement au dispositif de détermination de débit, au ou aux capteurs, aux moyens de stockage de données et aux moyens d'émission.
- les moyens de traitement sont aptes à et conçus pour traiter les valeurs de débit et de pression déterminées par ledit dispositif de détermination de débit et ledit ou lesdits capteurs et en déduire par exemple l'indice d'apnée/hypopnée, l'indice de troubles respiratoire ou Respiratory Disturbance Index (RDI), la pression de traitement moyenne, le débit moyen, et si le patient ronfle, le nombre d'événements respiratoires identifiés comme des limitation de débit...
- les moyens d'alimentation en courant électrique comprennent une alimentation électrique à tension inférieure à 50 V, par exemple une pile, une batterie rechargeable, une alimentation secteur avec transformateur de courant, typiquement à tension comprise entre 2 et 25 V.
- le système émetteur radiofréquence comprend un modem GSM ou GPRS intégré ou extérieur au boîtier commun.
- il comprend en outre un ou des indicateurs, telles des LEDs de couleurs, par exemple rouge et verte, fournissant à l'utilisateur une information relative à l'efficacité du traitement appliqué au moyen du dispositif.
- l'algorithme calcule au moins une donnée d'efficacité de traitement de l'OAS en déterminant, par périodes T (typiquement 15 minutes) le nombre d'événements détectés comme des apnées, des hypopnées ou des limitations de débit pendant cette période.
- pour déterminer le nombre de limitation de débit, d'hypopnée, d'apnée ou la durée de ronflement du patient par période T, l'algorithme :
   i) mesure la pression et le débit pendant la période de temps considérée,
   ii) en isole chaque respiration, pendant la période de temps, et regroupe les mesures de débits et de pression pour chaque respiration,
   iii) analyse les mesures et pour chaque groupe de mesures de pression et de débit, en reconnaissant le type de respiration pour déterminer un évènement de type apnée, hypopnée, limitation de débit ou ronflement, et
   iv) compte le nombre d'évènement détectés de type apnée, hypopnée, limitation de débit ou ronflement, pendant la période de temps considérée.
- le modem GSM ou GPRS envoie les enregistrements des données d'observance et d'efficacité du traitement du patient à un serveur permettant de générer des rapports d'observance et d'efficacité de traitement.

Selon un autre aspect, l'invention porte aussi sur un appareillage de traitement de l'AOS comprenant une machine de ventilation (M) reliée fluidiquement à l'entrée (7a) d'un dispositif selon l'invention, de manière à alimenter ledit dispositif en gaz sous pression.

Selon encore un autre aspect, l'invention concerne également un procédé pour déterminer au moins une durée de traitement journalier et au moins une donné d'efficacité de traitement de l'AOS, dans lequel :
a) on détermine au moyen d'un dispositif de détermination de débit, tel un capteur de débit,, le débit du gaz circulant entre l'entrée et la sortie du passage de gaz d'un dispositif selon l'invention relié fluidiquement à un appareil de traitement de l'AOS selon l'invention,
b) on détermine à l'aide de deux capteurs de pression absolue ou d'un capteur de pression relative, la différence de pression entre au moins une partie du passage et l'atmosphère extérieure,
c) on traite les valeurs de débit et de pression déterminées aux étapes a) et b), au moyen de moyens de traitement reliés au dispositif de détermination de débit et audit ou auxdits capteurs et on en déduit une donnée de durée de traitement journalier et une donné d'efficacité de traitement de l'AOS,
d) on mémorise dans des moyens de stockage de données, au moins l'une des données obtenues à l'étape c), et
e) on transmet, via des moyens d'émission, au moins l'une desdites données obtenues à l'étape c).

Selon le cas, le procédé de l'invention peut comprendre l'une ou plusieurs des caractéristiques suivantes :
- à l'étape e), la transmission d'au moins l'une des données obtenues à l'étape c) se fait à l'aide de moyens d'émission sans fil comprenant un système émetteur sans fil et une antenne
- à l'étape c), le traitement est opéré au moyen d'au moins un microcontrôleur et d'un algorithme.
- à l'étape d), le stockage de données se fait au moyen d'au moins une puce mémoire ou carte mémoire.

L'invention va être mieux comprise grâce à la description suivante, faite en références aux Figures annexées parmi lesquelles :
- la Figure 1 est un schéma de principe d'un mode de réalisation d'un dispositif selon la présente invention ;
- la Figure 2 est un schéma du principe de communication du dispositif de la Figure 1;
- la Figure 3 représente un algorithme de calcul de données d'observance utilisable dans le cadre de la présente invention,
- et la Figure 4 représente un algorithme de calcul de données d'efficacité utilisable dans le cadre de la présente invention.

Un dispositif de suivi de l'observance d'un traitement de l'AOS selon la présente invention est formé d'un boîtier B venant se raccorder sur le trajet du gaz respiratoire, typiquement de l'air sous pression, c'est-à-dire sur le circuit patient reliant la machine M de traitement de l'AOS au masque respiratoire, en général nasal, équipant le patient P à traiter.

La connexion du dispositif au circuit patient de la machine M se fait au moyen de tubes flexibles à embouts classiques, par exemple des embouts de diamètre égal à 22 mm et conformes à la norme ISO 5356-1.

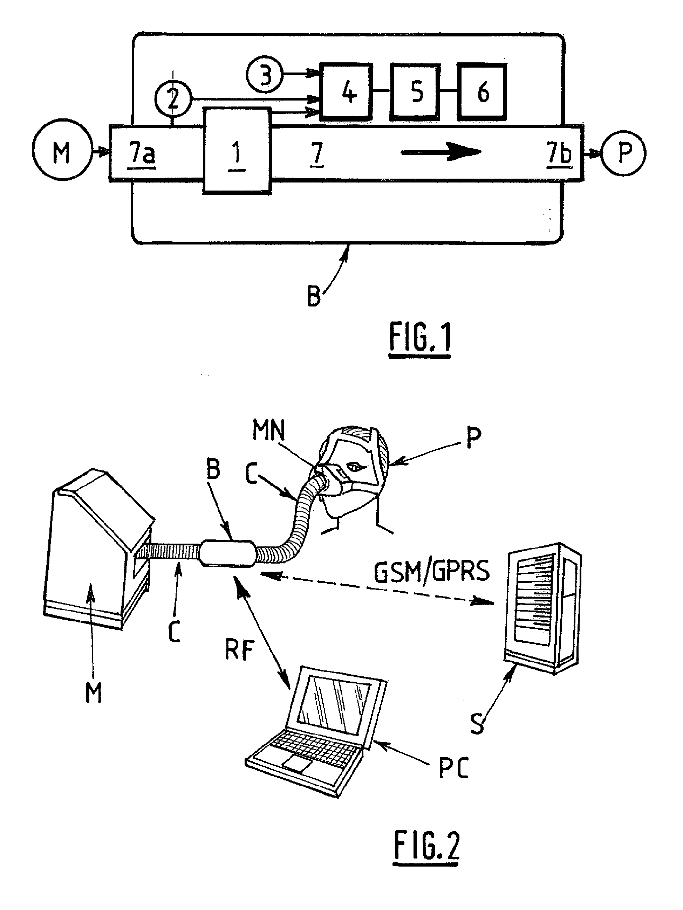

Comme illustré en Figure 1, le dispositif comporte un boîtier B avec un passage 7 interne de gaz avec une entrée 7a et une sortie 7b au sein duquel transite le gaz débité par l'appareil M de traitement de l'AOS, avant d'être envoyé au patient P.

Un dispositif de détermination de débit 1, couramment appelé « capteur de débit », est agencé dans le boîtier B et est relié au passage 7 de manière à permettre une mesure du débit du gaz circulant à l'intérieur dudit passage 7, c'est-à-dire entre l'entrée 7a et la sortie 7b du passage 7.

Par ailleurs, deux capteurs de pression absolue ou un capteur de pression relative 2, 3 sont également agencés sur ce passage 7 de manière à permettre une détermination la pression à l'intérieur du passage 7 entre lesdites entrée 7a et sortie 7b.

Lesdits capteurs de débit 1 (i.e. le dispositif de détermination de débit) et de pression 2, 3 sont connectés par ailleurs à des moyens de traitement 4, tel qu'un microcontrôleur à algorithme(s), par exemple le microcontrôleur MSP430 de Texas Instruments, capable de traiter les mesures de pression et de débit délivrées par les capteurs 1 à 3, afin d'en déduire la durée de traitement journalier et l'efficacité du traitement de l'AOS du patient P.

Le dispositif de détermination de débit 1 peut être un débitmètre à fil chaud ou un débitmètre déprimogène.

Par ailleurs, les capteurs de pression 2, 3 peuvent être par exemple des capteurs BMP085 commercialisés par la société BOSCH qui sont des capteurs de pression digitaux barométriques à haute précision (jusqu'à 0,03 hPa) et faible consommation en énergie (jusqu'à environ 3 µA).

Des moyens de stockage de données 5 sont utilisés pour mémoriser tout ou partie des données ainsi mesurées, par exemple une puce mémoire d'enregistrement de données ou une carte mémoire enfichable, notamment une carte mémoire flash de 8 Go de SST.

Par ailleurs, des moyens d'émission 6, tel un émetteur radiofréquence et son antenne, sont prévus pour permettre de transmettre, de préférence via une transmission sans fil, tout ou partie desdites données vers un récepteur situé à distance, tel un ordinateur ou un serveur, comme illustré sur la Figure 2. L'émetteur radiofréquence peut par exemple être équipé d'une antenne de 870 MHz de PHYCOMP.

Des moyens d'alimentation en courant électrique raccordés électriquement aux capteurs 1, 2, 3, aux moyens de stockage de données 5 et aux moyens d'émission assurent l'alimentation électrique du dispositif, par exemple une alimentation électrique à basse tension comprenant une ou plusieurs batteries, piles...

Le dispositif selon la présente invention a pour fonction de mesurer, indépendamment de la machine M de traitement de l'AOS et donc sans utiliser d'information ou de donnée interne à cet appareil, et de communiquer à distance, sans fil, les informations relatives non seulement à l'observance mais également à l'efficacité d'un traitement par PPC, c'est-à-dire la durée de traitement effective des patients, ainsi que les évènements notamment les apnées, hypopnées, limitations de débit, ronflements, fuites...

Ceci permet d'obtenir un suivi quotidien et une sécurité dans le suivi du traitement du patient à domicile grâce à la possibilité d'alarmer le patient et son centre de soins en cas de non suivi de la prescription ou de non efficacité du traitement.

Comme expliqué ci-avant et illustré en Figure 2, le boîtier B du dispositif selon la présente invention vient s'incorporer sur le trajet du gaz, c'est-à-dire la ou les conduites C flexibles d'acheminement de gaz, entre l'appareil M distribuant le gaz sous pression positive continue et le patient P équipé d'un masque nasal ou facial MN, et permet de mesurer et enregistrer le temps de traitement journalier, ainsi que l'efficacité du traitement.

Il est conçu pour pouvoir s'adapter à tout type de machine M de traitement du SAOS, c'est-à-dire les ventilateurs de types CPAP, APAP, BiPAP et analogues.

Le dispositif de l'invention possède une capacité d'enregistrement de plusieurs mois, de préférence d'au moins 1 an, laquelle peut être encore étendue.

Il peut communiquer les données enregistrées par liaison filaire USB vers un ordinateur, un PDA, un serveur ou tout autre moyen capable d'enregistrer directement les données transmises, comme montré en Figure 2. En fait, le dispositif transmet à distance à l'aide du modem GSM ou GPRS intégré qui envoie les enregistrements de données d'observance et d'efficacité du traitement du patient jusqu'au centre de soins par exemple, où un serveur S adapté permet de générer des rapports d'observance et d'efficacité de traitement.

Comme déjà dit, le dispositif de l'invention est de mesurer la durée de traitement du patient et son efficacité lorsqu'il se traite. En fait, un patient est considéré comme étant bien en cours de traitement si deux conditions sont remplies, à savoir :
- si l'on détecte la respiration du patient par un algorithme spécifique de traitement des signaux de débit et de pression mesurés dans le circuit patient permettant de déduire de ces signaux si le patient porte bien le masque nasal ou naso-buccal ; et
- si l'on détecte une pression minimale de traitement correspondant à une machine PPC qui est en marche et qui fonctionne correctement.

Ces deux conditions peuvent être déterminées à l'aide de mesures de pression et de débit dans le circuit patient grâce au dispositif de l'invention équipé d'un capteur de débit et soit de deux capteurs de pression absolue, à savoir l'un pour mesurer la pression dans le passage 7, l'autre pour mesurer la pression atmosphérique, soit d'un capteur de pression relative. Un tel agencement permet de déduire les variations de pression et de débit liées, d'une part, à la délivrance d'air et, d'autre part, aux inspirations et expiration du patient dans le circuit patient.

L'efficacité du traitement est alors déduite des variations de pression et de débit liées aux inspirations/expirations du patient et du niveau de pression de traitement. Elle est mesurée par la détection du nombre d'apnées, d'hypopnées, de limitations de débit, de fuite du circuit patient ou du masque, et du temps de ronflement survenant pendant le traitement.

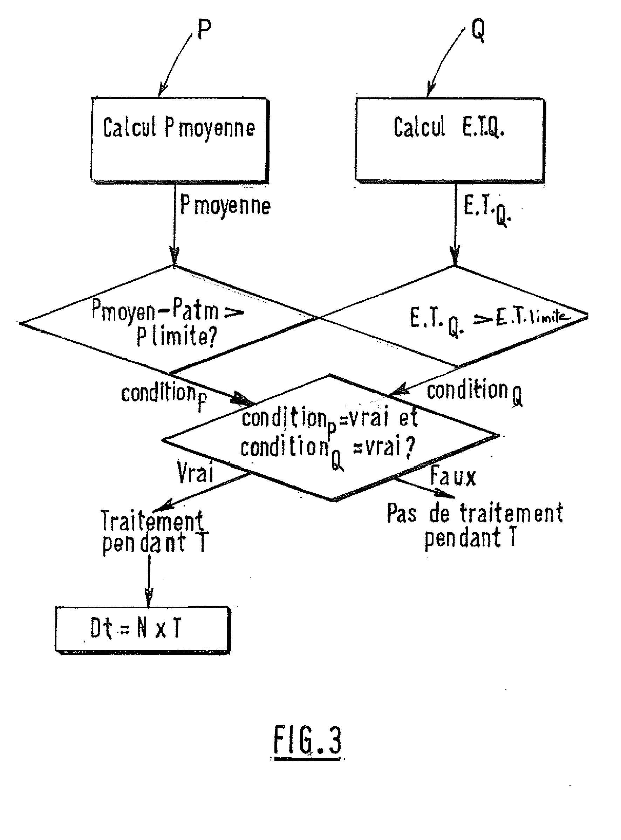

La figure 3 représente un algorithme de calcul de données d'observance applicable dans le cadre de la présente invention.

Pour calculer la durée de suivi du traitement par un patient donné, le temps est divisé en périodes d'une durée fixée que l'on appelle T, typiquement des périodes de 1 minute par exemple.

Pour chacune de ces périodes T, l'algorithme détermine si pendant cette période T, le patient suit son traitement, puis il compte le nombre de périodes pendant lesquelles le patient a suivi son traitement et calcule ainsi la durée de suivi du traitement.

A partir des mesures de pression P et de débit Q prises dans le dispositif, l'algorithme vérifie sur une période donnée, deux conditions (conditionP et conditionQ) qui sont nécessaires et suffisantes pour déterminer si pendant cette période le traitement a été suivi, à savoir :
- une condition sur la pression : conditionP. La Pmoyenne est d'abord calculée. Si la pression Pmoyenne dans le circuit du patient est suffisamment surélevée par rapport à la pression atmosphérique Patm, alors le circuit du patient est branché sur l'appareil de traitement de l'OAS et ce dernier fonctionne, c'est-à-dire qu'il délivre une pression positive d'au moins 4 cm H₂O, donc supérieure à une valeur de pression minimum préfixée P.limite.
- une condition sur le débit : conditionQ. Si l'écart-type du signal de débit E.T.Q calculé dépasse un certain seuil fixé E.T.limite, alors les mesures de débit varient avec une amplitude qui ne peut être due qu'à la respiration du patient dans le masque.

En fonction des résultats du calcul des conditionP et conditionQ, on déduit si le traitement a bien eu lieu ou non pendant la période de temps T considérée et, dans l'affirmative, la durée du traitement Dt qui correspond au nombre N de périodes de traitement multiplié par la durée de la période T.

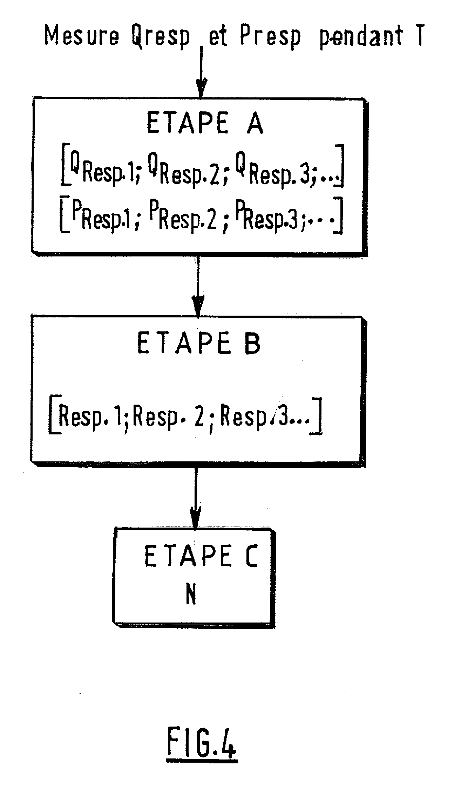

De façon analogue, la Figure 4 représente un algorithme de calcul de données d'efficacité applicable dans le cadre de la présente invention.

Dans ce cas, pour calculer les données d'efficacité, il faut déterminer pour chaque cycle respiratoire du patient, comprenant la phase d'inspiration et celle d'expiration du patient, si cette respiration correspond à une limitation de débit, à une hypopnée, ou à une apnée, et si pendant cette respiration le patient a ronflé.

Pour ce faire, on mesure d'abord les pressions Presp et débit Qresp pendant la période T considérée.

L'algorithme doit ensuite (étape A) isoler chaque respiration, pendant la période T, et regrouper les mesures de débits (Qresp.1, Qresp.2,... Qresp.i) et de pression (Presp.1, Presp.2,... Presp.i) pour chaque respiration. Pour se faire, l'algorithme suit 3 étapes :
- il estime le niveau de base du signal de débit. Ce niveau de base correspond au débit mesuré à la fin d'une expiration. Il s'agit en fait du débit lorsque le patient n'est ni en train d'inspirer ni en train d'expirer : c'est aussi le débit de fuite intentionnelle du masque. Plusieurs estimateurs peuvent être utilisés pour le calculer : une moyenne glissante, une médiane glissante, et d'autres plus sophistiqués basés sur le calcul du débit de fuite intentionnelle du masque
- ensuite, l'algorithme détecte les instants où le débit instantané passe de en-dessous à au-dessus du niveau de base estimé. Ces instants correspondent au début des inspirations.
- il enregistre alors les signaux de débit Qresp.i et de pression Presp.i depuis le dernier instant détecté comme le début d'une inspiration : on a donc les signaux de débit et de pression entre deux débuts d'inspiration c'est-à-dire pendant une respiration.

Ensuite, à l'étape B, on analyse les mesures et pour chaque groupe de mesures Presp.i et Qresp.i, on reconnaît le type de respiration Resp.i pour déterminer une apnée, une hypopnée, une limitation de débit et/ou des ronflements. Chaque respiration doit être étiquetée comme normale, limitation de débit, hypopnée ou apnée, et elle doit avoir une étiquette avec ou sans ronflements. Pour déterminer ces étiquettes l'algorithme suit la procédure suivante :
- il commence par caractériser une respiration normale. Il caractérise sa durée, son volume, son amplitude, la forme du signal de débit à l'inspiration (l'algorithme peut utiliser pour cela des niveau de corrélations avec des formes données comme par exemple un sinus, ou bien des valeurs des dérivées pendant l'inspiration ou autre) et le niveau de bruit aux hautes fréquences. Pour cela, il se base sur un historique de respiration qu'il a gardé en mémoire et il calcule ces paramètres caractéristiques pour chacune des respirations de l'historique. Puis, il utilise un estimateur du type moyenne, médiane ou plus sophistiqué pour évaluer la valeur de ces paramètres pour une respiration normale. L'historique doit être suffisant pour contenir au moins une respiration normale, typiquement entre 2 et 5 minutes.
- Ensuite il compare les paramètres de chaque respiration à la référence estimée précédemment et effectue le classement suivant :
   - Si l'inspiration est inférieure à 20% d'une inspiration normale (en volume ou en amplitude, ou autre) alors la respiration est étiquetée comme une apnée.
   - Sinon, si elle est inférieure à 50% d'une inspiration normale alors la respiration est étiquetée comme une hypopnée.
   - Sinon, si la forme de l'inspiration est différente de la forme d'une inspiration normale, et caractéristique d'une limitation de débit alors la respiration est étiquetée comme étant une limitation de débit.
   - Sinon la respiration est étiquetée comme une respiration normale.
- Puis il estime le niveau de bruit aux hautes fréquences pendant l'inspiration. Il le compare au niveau de bruit aux hautes fréquences normale : s'il est supérieur alors la respiration est étiquetée comme étant avec du ronflement sinon elle est étiquetée comme étant sans ronflement.

Enfin, pendant l'étape C, l'algorithme compte le nombre N d'évènement détectés de type apnée, hypopnée, limitation de débit, ronflements, ou autres, pendant la période T. Pour les évènements de type apnée, hypopnée ou limitation de débit, l'algorithme étudie un historique glissant des paramètres caractéristiques des dernières respirations. Il analyse les ensembles consécutifs de respirations étiquetées comme non-normales. Si un ensemble de respirations dure plus de dix secondes, l'algorithme comptabilise un nouvel évènement. Le choix de le comptabiliser comme une apnée, une hypopnée ou une limitation de débit est fait en fonction de critères sur les paramètres caractéristiques de chaque respiration, sur l'enchainement de respirations ayant la même étiquette, et sur les proportions de respirations avec une étiquette donnée par rapport aux autres. Ces critères ont été mis au point par apprentissage sur des données de test. L'apprentissage (par exemple un apprentissage statistique supervisé comme l'analyse discriminante) a été réalisé en prenant comme étiquette de référence l'étiquette des évènements simulés (pour les données de tests faits en simulation) ou l'étiquette des évènements déterminée par un technicien spécialisé d'un laboratoire du sommeil (pour les données de tests réels).

Pour les ronflements, l'algorithme comptabilise une période T, par exemple 1 min, de ronflement si pendant au moins 50% de T les respirations ont été étiquetées avec ronflement.

En outre, en ce qui concerne les fuites, l'algorithme compare le débit mesuré au débit que l'on devrait mesurer étant donné la pression de fonctionnement et le masque utilisé. Il en déduit alors une présence ou absence de fuites de gaz.

D'une façon générale, le traitement par pression positive continue (PPC) est un traitement efficace pour les patients atteints du syndrome d'apnée obstructive du sommeil ou SAOS mais les effets du traitement par PPC sont négligeables, voire nuls, si le patient n'observe pas correctement son traitement, c'est-à-dire pendant une durée inférieure à 4 heures par nuit.

Connaître l'observance des patients, c'est-à-dire mesurer le temps réel pendant lequel ils suivent leur traitement est donc essentiel dans l'étude de cette maladie et des soins qui leur sont associés.

Dès lors, la possibilité de suivre quotidiennement l'efficacité du traitement prescrit permet au médecin traitant d'ajuster sa prescription dès les premières semaines de traitement.

De même, le suivi de l'observance permet au médecin d'apprécier précocement l'acceptation du traitement par le patient.

Le dispositif de l'invention permet une telle observance et donc un suivi efficace des patients grâce au traitement des données mesurées par un algorithme indépendant de la machine de traitement du SAOS. La délivrance automatique des résultats d'observance et d'efficacité du traitement est opérée quotidiennement et sans déplacement d'un intervenant puisque tout se fait à distance. Ensuite, l'interprétation des rapports est aisée et ce, quelle que soit la machine de traitement utilisée.

Le dispositif de l'invention est utilisable dans le cadre de toute méthode de traitement thérapeutique du syndrome d'apnée obstructive du sommeil (SAOS) d'un patient se caractérisant notamment par une obstruction de ses voies aériennes supérieures.

## Revendications

1. Dispositif de suivi de l'observance d'un traitement de l'AOS comprenant :
- un passage (7) de gaz comprenant une entrée (7a) et une sortie (7b), auquel est raccordé un dispositif de détermination de débit (1) pour déterminer le débit du gaz circulant entre l'entrée (7a) et la sortie (7b),
- deux capteurs de pression absolue ou un capteur de pression relative (2, 3) pour déterminer la différence de pression entre au moins une partie du passage (7) et l'atmosphère extérieure,
- des moyens de stockage de données (5) pour mémoriser au moins l'une desdites données,
- des moyens d'émission (6) aptes à et conçus pour transmettre au moins l'une desdites données, et
- au moins la portion de passage (7) de gaz située entre l'entrée (7a) et la sortie (7b), les moyens de stockage de données (5) et les moyens d'émission sont compris dans un boîtier,
**caractérisé en ce que** :
- il comprend des moyens de traitement (4) comprennent au moins un microcontrôleur et un algorithme,
- ledit dispositif de détermination de débit (1) et ledit ou lesdits capteurs de pression (2, 3) sont reliés aux moyens de traitement (4),
- lesdits moyens de traitement (4) étant aptes à et conçus pour :
i) traiter les valeurs de débit et de pression déterminées par ledit dispositif de détermination de débit (1) et ledit ou lesdits capteurs (2, 3) et en déduire au moins une donnée de durée de traitement journalier et au moins une donnée d'efficacité de traitement de l'AOS, où :
. ladite donnée d'efficacité de traitement étant déterminée à partir du nombre d'évènements respiratoires détectés choisis parmi les apnées, les hypopnées, les limitations de débit et les ronflements, pendant une période de temps T, par l'algorithme des moyens de traitement (4) à partir des mesures de pression et de débit opérées pendant la période de temps T considérée, et
. ladite durée de traitement étant déterminée par l'algorithme, pour un temps donné divisé en périodes d'une durée fixée (T) en :
a) déterminant si, pendant une partie des périodes de temps (T) données, le patient suit son traitement,
b) comptabilisant le nombre de périodes pendant lesquelles le patient a suivi son traitement et
c) calculant la durée de suivi du traitement, ladit durée du traitement (Dt) correspondant au nombre (N) de périodes de traitement multiplié par la durée de la période (T) considérée,
ii) détecter la respiration du patient et la pression minimale de traitement par traitement des signaux de débit (Q) et de pression (P) mesurés par ledit dispositif de détermination de débit (1) et ledit ou lesdits capteurs de pression (2, 3), l'algorithme vérifiant sur une période de temps donnée, à partir des mesures de pression (P) et de débit (Q), si la pression (Pmoyenne) dans le circuit du patient est suffisamment élevée par rapport à la pression atmosphérique (Patm), et si l'écart-type du signal de débit (E.T.Q) dépasse un seuil fixé (E.T.limite), et en déduisant si le traitement a eu lieu ou non, pendant la période de temps (T) considérée,
- et les moyens d'émission (6) comprennent un système émetteur sans fil et une antenne permettant d'assurer une transmission sans fil des données.

2. Dispositif de suivi selon la revendication 1, **caractérisé en ce que**, pour déterminer si la respiration durant chaque cycle respiratoire correspond à une limitation de débit, à une hypopnée, à une apnée ou à un ronflement du patient, l'algorithme :
i) mesure la pression (Presp) et le débit (Qresp) pendant la période (T) considérée,
ii) en isole chaque respiration, pendant la période T, et regroupe les mesures de débits (Qresp.1, Qresp.2,... Qresp.i) et de pression (Presp.1, Presp.2,... Presp.i) pour chaque respiration,
iii) analyse les mesures et pour chaque groupe de mesures de pression et de débit (Presp.i ; Qresp.i), en reconnaissant le type de respiration (Resp.i) pour déterminer un évènement de type apnée, hypopnée, limitation de débit ou ronflement, et
iv) compte le nombre (N) d'évènement détectés de type apnée, hypopnée, limitation de débit ou ronflement, pendant la période T.

3. Dispositif de suivi selon l'une des revendications précédentes, **caractérisé en ce que** les moyens de stockage de données (5) comprennent au moins une puce mémoire ou une carte mémoire.

4. Dispositif de suivi selon l'une des revendications précédentes, **caractérisé en ce que** le système émetteur radiofréquence comprend un modem GSM ou GPRS.

5. Dispositif de suivi selon l'une des revendications précédentes, **caractérisé en ce que** le modem GSM ou GPRS envoie les enregistrements des données d'observance et d'efficacité du traitement du patient à un serveur (S) permettant de générer des rapports d'observance et d'efficacité de traitement.

6. Dispositif de suivi selon l'une des revendications précédentes, **caractérisé en ce qu'**il comprend en outre des moyens d'alimentation en courant électrique raccordés électriquement au dispositif de détermination de débit (1) et audit ou auxdits capteurs de pression (2, 3), aux moyens de stockage de données (5) et aux moyens d'émission (6).

7. Dispositif de suivi selon l'une des revendications précédentes, **caractérisé en ce que** les moyens d'alimentation en courant électrique comprennent une alimentation électrique à tension inférieure à 50V.

8. Dispositif de suivi selon l'une des revendications précédentes, **caractérisé en ce qu'**il comprend en outre un ou des indicateurs fournissant à l'utilisateur (P) une information relative à l'efficacité du traitement appliqué au moyen du dispositif.

9. Appareillage de traitement de l'AOS comprenant une machine de ventilation (M) reliée fluidiquement à l'entrée (7a) d'un dispositif selon l'une des revendications précédentes, de manière à alimenter ledit dispositif en gaz sous pression.

## Patentansprüche

1. Vorrichtung zur Verfolgung der Einhaltung einer Behandlung der OSA, umfassend:
- einen Gasdurchgang (7), umfassend einen Eingang (7a) und einen Ausgang (7b), mit dem eine Vorrichtung zur Bestimmung des Durchsatzes (1) verbunden ist, um den Durchsatz des Gases zu bestimmen, der zwischen dem Eingang (7a) und dem Ausgang (7b) zirkuliert,
- zwei Sensoren des absoluten Drucks oder einen Sensor des relativen Drucks (2, 3), um den Druckunterschied zwischen mindestens einem Teil des Durchgangs (7) und der äußeren Atmosphäre zu bestimmen,
- Mittel zum Speichern von Daten (5), um mindestens eines der Daten aufzuzeichnen,
- Sendemittel (6), die dazu geeignet und entworfen sind, um mindestens eines der Daten zu übertragen, und
- mindestens den Abschnitt des Gasdurchgangs (7), der sich zwischen dem Eingang (7a) und dem Ausgang (7b) befindet, wobei die Mittel zum Speichern von Daten (5) und die Mittel zum Senden von Daten in einem Gehäuse enthalten sind,
**dadurch gekennzeichnet, dass**:
sie Behandlungsmittel (4) umfasst, die mindestens eine Mikrosteuerungsvorrichtung und einen Algorithmus umfassen,
- wobei die Vorrichtung zur Bestimmung des Durchsatzes (1) und der oder die Sensor (en) des Drucks (2, 3) mit den Behandlungsmitteln (4) verbunden sind,
- wobei die Behandlungsmittel (4) dazu geeignet und entworfen sind, um:
i) die Werte des Durchsatzes und des Drucks, bestimmt durch die Vorrichtung zur Bestimmung des Durchsatzes (1) und des oder der Sensors/Sensoren (2, 3) zu verarbeiten und davon mindestens ein Datum der Dauer der täglichen Behandlung und mindestens ein Datum der Wirksamkeit der Behandlung der OSA abzuleiten, wobei
- das Datum der Wirksamkeit der Behandlung auf der Grundlage der Anzahl von nachgewiesenen respiratorischen Ereignissen, ausgewählt aus den Apnoen, den Hypopnoen, den Begrenzungen des Durchsatzes und dem Schnarchen, während eines Zeitraums T, durch den Algorithmus der Behandlungsmittel (4) auf der Grundlage der Messungen des Drucks und des Durchsatzes bestimmt wird, die sich während des in Betracht gezogenen Zeitraums T ergeben, und
- wobei die Dauer der Behandlung durch den Algorithmus für eine gegebene Zeit, unterteilt in Zeiträume mit einer festen Dauer (T) bestimmt wird durch:
a) die Bestimmung ob, während eines Teils der bestimmten Zeiträume (T), der Patient seine Behandlung einhält,
b) die Zählung der Anzahl von Zeiträumen, während der der Patient seine Behandlung eingehalten hat, und
c) die Berechnung der Dauer der Einhaltung der Behandlung, wobei die Dauer der Behandlung (Dt) der Anzahl (N) von Behandlungszeiträumen entspricht, multipliziert durch die Dauer des in Betracht gezogenen Zeitraums (T),
ii) die Atmung des Patienten und den minimalen Druck der Behandlung durch die Verarbeitung der Signale des Durchsatzes (Q) und des Drucks (P), gemessen durch die Vorrichtung zur Bestimmung des Durchsatzes (1) und den oder die Sensor(en) (2, 3) nachzuweisen, wobei der Algorithmus für einen gegebenen Zeitraum auf der Grundlage der Messungen des Drucks (P) und des Durchsatzes (Q) überprüft, ob der Druck (Pmoyenne) im Kreislauf des Patienten mit Bezug auf den atmosphärischen Druck (Patm) hoch genug ist, und ob die typische Abweichung des Signals des Durchsatzes (E.T.Q) eine feste Schwelle (E.T.limite) übersteigt, und Folgerung daraus, ob die Behandlung während des in Betracht gezogenen Zeitraums (T) stattgefunden hat oder nicht,
- und die Sendemittel (6), ein drahtloses Sendesystem und eine Antenne umfassen, die ermöglichen, eine drahtlose Übertragung der Daten sicherzustellen.

2. Vorrichtung zur Verfolgung nach Anspruch 1, **dadurch gekennzeichnet dass**, um zu bestimmen, ob die Atmung während jedes respiratorischen Zyklus einer Begrenzung des Durchsatzes einer Hypopnoe, einer Apnoe oder einem Schnarchen des Patienten entspricht, der Algorithmus:
i) den Druck (Presp) und den Durchsatz (Qresp) während des in Betracht gezogenen Zeitraums (T) misst,
ii) davon jede Atmung während des Zeitraums T isoliert und die Messungen der Durchsätze (Qresp.1, Qresp.2,...Qresp.i) und des Drucks (Presp.1, Presp.2,... Presp.i) für jede Atmung neu gruppiert,
iii) die Messungen für jede Gruppe von Messungen des Druck und des Durchsatzes (Presp.i; Qresp.i) analysiert, indem die Art der Atmung (Resp.i) erkannt wird, um eine Ereignis vom Typ Apnoe, Hypopnoe, Begrenzung des Durchsatzes oder Schnarchen zu bestimmen, und
iv) die Anzahl (N) der nachgewiesenen Ereignisse vom Typ Apnoe, Hypopnoe, Begrenzung des Durchsatzes oder Schnarchen während des Zeitraums T zählt.

3. Vorrichtung zur Verfolgung nach einem der vorhergehenden Ansprüche, **dadurch gekennzeichnet, dass** die Mittel zur Speicherung der Daten (5) mindestens einen Speicherchip oder eine Speicherkarte umfassen.

4. Vorrichtung zur Verfolgung nach einem der vorhergehenden Ansprüche, **dadurch gekennzeichnet, dass** das Funkfrequenz-Sendesystem ein GSM- oder GPRS-Modem umfasst.

5. Vorrichtung zur Verfolgung nach einem der vorhergehenden Ansprüche, **dadurch gekennzeichnet, dass** das GSM- oder GPRS-Modem die Aufzeichnungen der Daten der Beobachtung und der Wirksamkeit der Behandlung des Patienten an einen Server (S) schickt, der ermöglicht, Berichte der Beobachtung- und Wirksamkeit der Behandlung zu erzeugen.

6. Vorrichtung zur Verfolgung nach einem der vorhergehenden Ansprüche, **dadurch gekennzeichnet, dass** sie außerdem Mittel zur Versorgung mit elektrischem Strom umfasst, die elektrisch mit der Vorrichtung zur Bestimmung des Durchsatzes (1) und mit dem oder den Drucksensor(en) (2, 3), mit den Mitteln zur Speicherung von Daten (5) und mit Sendemitteln (6) verbunden sind.

7. Vorrichtung zur Verfolgung nach einem der vorhergehenden Ansprüche, **dadurch gekennzeichnet, dass** die Mittel zur Versorgung mit elektrischem Strom eine elektrische Versorgung mit einer Spannung von weniger als 50V umfassen.

8. Vorrichtung zur Verfolgung nach einem der vorhergehenden Ansprüche, **dadurch gekennzeichnet, dass** sie außerdem eine oder mehrere Anzeigen umfasst, die dem Benutzer (P) eine Information mit Bezug auf die Wirksamkeit der Behandlung liefert, die mit Hilfe der Vorrichtung angewendet wird.

9. Gerät zur Behandlung von OSA, umfassend eine Ventilationsmaschine (M), die fluidisch mit dem Eingang (7a) einer Vorrichtung nach einem der vorhergehenden Ansprüche verbunden ist, um die Vorrichtung mit Gas unter Druck zu versorgen.

## Claims

1. Device for monitoring OSA treatment compliance comprising:
- a gas duct (7) comprising an inlet (7a) and an outlet (7b), to which a flow rate determination device (1) is connected to determine the flow rate of the gas circulating between the inlet (7a) and the outlet (7b),
- two absolute pressure sensors or a relative pressure sensor (2, 3) to determine the pressure differential between at least a portion of the duct (7) and the external atmosphere,
- data storage means (5) to save at least one of said data items in memory,
- transmission means (6) suitable and designed for transmitting at least one of said data items, and
- at least one gas duct (7) portion situated between the inlet (7a) and the outlet (7b), the data storage means (5) and the transmission means are included in a housing,
**characterised in that**:
- it comprises processing means (4) comprising at least one microcontroller and an algorithm,
- said flow determination device (1) and said pressure sensor(s) (2, 3) are connected to the processing means (4),
- said processing means (4) being suitable and designed for:
i) processing the flow rate and pressure values determined by said flow rate determination device (1) and said sensor (s) (2, 3) and inferring at least one daily treatment time data item and at least one OSA treatment efficacy data item, where:
. said treatment efficacy data item being determined on the basis of the number of respiratory events detected chosen from apnoea, hypnoea, flow restrictions and snoring, during a time period T, by the algorithm of the processing means (4) on the basis of the pressure and flow rate measurements made during the time period T in question, and
. said treatment time being determined by the algorithm, for a given time divided into periods of fixed duration (T) by:
a) determining whether, during a portion of the given time periods (T), the patient complies with the treatment,
b) counting the number of periods during which the patient has complied with the treatment and
c) calculating the treatment compliance time, said treatment time (Dt) corresponding to the number (N) of treatment periods multiplied by the duration of the period (T) in question,
ii) detecting the patient's breathing and the minimum treatment pressure by processing the flow rate (Q) and pressure (P) signals measured by said flow determination device (1) and said pressure sensor(s) (2, 3), the algorithm checking over a given time period, on the basis of the pressure (P) and flow rate (Q) measurements, whether the pressure (Pmean) in the patient circuit is sufficiently high with respect to the atmospheric pressure (Patm), and whether the standard deviation of the flow rate signal (S.D.Q) exceeds a set threshold (S.D.limit), and by inferring whether the treatment took place or not, during the time period (T) in question,
- and the transmission means (6) comprise a wireless transmitter system and an antenna enabling wireless data transmission.

2. Monitoring device according to claim 1, **characterised in that**, to determine whether the breathing during each respiratory cycle corresponds to flow restriction, hypopnoea, apnoea or snoring on the part of the patient, the algorithm:
i) measures the pressure (Presp) and the flow rate (Qresp) during the period (T) in question,
ii) isolates each breath therefrom, during the period T, and groups the flow rate (Qresp.1, Qresp.2, ... Qresp.i) and pressure (Presp.1, Presp.2, ... Presp.i) measurement for each breath,
iii) analyses the measurements and for each group of pressure and flow rate measurements (Presp.i; Qresp.i), by recognising the type of breath (Resp.i) to determine an event such as apnoea, hypopnoea, flow restriction or snoring, and
iv) counts the number (N) of events detected such as apnoea, hypopnoea, flow restriction or snoring, during the period T.

3. Monitoring device according to any of the above claims, **characterised in that** the data storage means (5) comprises at least one memory chip and one memory card.

4. Monitoring device according to any of the above claims, **characterised in that** the radiofrequency transmitter system comprises a GSM or GPRS modem.

5. Monitoring device according to any of the above claims, **characterised in that** the GSM or GPRS modem sends treatment compliance and efficacy data records in respect of the patient to a server (S) suitable for generating treatment compliance and efficacy reports.

6. Monitoring device according to any of the above claims, **characterised in that** it further comprises electricity power supply means electrically connected to the flow rate determination device (1) and to said pressure sensor (s) (2, 3), to the data storage means (5) and to the transmission means (6).

7. Monitoring device according to any of the above claims, **characterised in that** electricity power supply means comprise an electrical power supply having a voltage less than 50V.

8. Monitoring device according to any of the above claims, **characterised in that** it further comprises one or more indicators providing the user (P) with information relative to the efficacy of the treatment applied by means of the device.

9. OSA treatment apparatus comprising a ventilation machine (M) fluidically connected to the inlet (7a) of a device according to any of the above claims, so as to supply said device with pressurised gas.
